# EUROPEAN PATENT APPLICATION

(11) **EP 2 246 327 A1**
(43) Date of publication of application: **03.11.2010**
(21) Application number: 10158778.0
(22) Date of filing: 31.03.2010
(51) Int. Cl.: C07C 403/24, A61P 27/02, A61K 31/047

(54) **A process for isolation of lutein and zeaxanthin crystals from plant sources**

(30) Priority: 27.04.2009 IN CH09642009
(71) Applicant: Katra Phytochem (India) Private Limited, Bangalore 562107 KRN (IN)
(72) Inventor: Sethuraman, Swaminathan, 562107 Bangalore Dist., Karnataka (IN); Madavalappil, Kunhiraman, Priya, 562107 Bangalore Dist., Karnataka (IN)
(74) Representative: Dilg, Haeusler, Schindelmann Patentanwaltsgesellschaft mbH

(57) **Abstract**

The present invention relates to a process for the isolation of carotenoid crystals having lutein and zeaxanthin in a weight ratio of 10:1, 5:1 and 1:1, respectively. The process comprises contacting a plant source rich in lutein with hexane and extracting at a temperature of 40°C to 60°C to obtain an oleoresin rich in lutein; contacting a plant source rich in zeaxanthin with hexane and extracting at a temperature of 40°C to 60°C to obtain an oleoresin rich in zeaxanthin. The oleoresin rich in lutein and the oleoresin rich in zeaxanthin are mixed separately in a ratios ranging from 80:20(w/w) to 90:10(w/w) or 70:30(w/w) to 30:70(w/w) or 10:90(w/w) to 20:80(w/w), respectively, and homogenized to obtain a mixed oleoresin. The mixed oleoresin is hydrolyzed with an alcoholic alkali at a temperature of 70°C to 80°C to obtain a reaction mixture. The carotenoid crystals are precipitated by adding hot water to the reaction mixture to form a precipitate. Carotenoid crystals having lutein and zeaxanthin in a weight ratio of 10:1 1 or 5:1 or 1: 1, respectively, are obtained by filtering, washing, and drying the precipitate.

## Description

### FIELD OF THE INVENTION

The present invention relates to pure carotenoid crystals derived from plant sources, and particularly to their isolation and purification process.

### BACKGROUND OF THE INVENTION

Pure carotenoid crystals derived from marigold flowers, comprising predominantly of xanthophylls such as lutein, zeaxanthin, and cryptoxanthin and low levels of β -carotene have been proven scientifically to reduce the risk of age related macular degeneration (Moeller, S.M., Jacques, P.F., and Blumberg, J.B. "The potential role of dietary Xanthophylls in cataract and age related macular degeneration" Journal of the American College of Nutrition, 2000, 19: 522S-527S), control over LDL cholesterol (Chopra, M., and Thurnham, D., "Effect of lutein on oxidation of low density lipoproteins (LDL) in vitro", Proceedings of the Nutrition Society, 1994, 53: 1993, #18A.), prevention of Coronary heart diseases (Howard, A.N., Williams, N.R., Palmer, C.R., Cambou, J.P., Evans, A.E., Foote, J.W., et al., "Do hydroxy-carotenoids prevent coronary heart disease? A comparison between Belfast and Toulouse" International Journal of Vitamin and Nutrition Research, 1996, 66: 113-118), and free radicals scavenging and immunity enhancing (Chew, B.P., Wong, M.W., and Wong, T.S., "Effects of lutein from marigold extract on immunity and growth of mammary tumors in mice," Anticancer Research, 1996, 16: 3689-3694).

Lutein (β-ε-carotene-3-3'-diol) and zeaxanthin (β-β-carotene-3-3'-diol) belong to xanthophylls group in the carotenoid family with highly reactive hydroxyl groups which cannot be synthesized by humans and animals.

Carotenoids are a class of natural fat-soluble pigments found principally in plants, algae, and photosynthetic bacteria, where they play a critical role in the photosynthetic process. They also occur in some non-photosynthetic bacteria, yeasts, and molds, where they may carry out a protective function against damage by light and oxygen. Although animals appear to be incapable of synthesizing carotenoids, many animals incorporate carotenoids from their diet. Within animals, carotenoids provide bright coloration, serve as antioxidants, and can be a source for vitamin A activity (Ong and Tee 1992; Britton et al. 1995).

Carotenoids are responsible for many of the red, orange, and yellow hues of plant leaves, fruits, and flowers, as well as the colors of some birds, insects, fish, and crustaceans. Some familiar examples of carotenoid coloration are the oranges of carrots and citrus fruits, the reds of peppers and tomatoes, and the pinks of flamingoes and salmon (Pfander 1992). Some 600 different carotenoids are known to occur naturally (Ong and Tee 1992), and new carotenoids continue to be identified (Mercadante 1999).

Carotenoids are defined by their chemical structure. The majority of carotenoids are derived from a 40-carbon polyene chain, which could be considered the backbone of the molecule. This chain may be terminated by cyclic end-groups (rings) and may be complemented with oxygen- containing functional groups. The hydrocarbon carotenoids are known as carotenes, while oxygenated derivatives of these hydrocarbons are known as xanthophylls. Beta-carotene, the principal carotenoid in carrots, is a familiar carotene, while lutein, the major yellow pigment of marigold petals, is a common xanthophyll.

The structure of a carotenoid ultimately determines what potential biological function(s) that pigment may have. The distinctive pattern of alternating single and double bonds in the polyene backbone of carotenoids is what allows them to absorb excess energy from other molecules, while the nature of the specific end groups on carotenoids may influence their polarity.

The former may account for the antioxidant properties of biological carotenoids, while the latter may explain the differences in the ways that individual carotenoids interact with biological membranes (Britton 1995).

U.S. Patent No. 5,382,714 discloses process for isolation of pure lutein comprising from saponified marigold oleoresin containing free lutein.

U.S. Patent No. 5,648,564 uses aqueous alkali and propylene glycol wherein the carotenoid esters are neither soluble nor freely miscible with them and hence it requires very long time at higher temperature for the fatty esters to saponify which may result in exposure of the product for a longer duration under heat and air, promoting the formation of oxidative degenerative products and the process time is too long for a commercial batch.

U.S. Patent No. 6,743,953 describes final purification step involving multiple solvents like ethyl acetate, hexane, acetone and methanol with the possibilities of leaving residues of the same. Again, the process involves saponification up to 3 hrs by subjecting the product to heat at 70°C for more time which may result in degenerated oxidative products in the saponified mass.

U. S Patent No. 6,380,442 states that the hydrolysis of carotenoids is done by using isopropyl alcohol with saponification time being 90 minutes.

U.S. Patent No. 6,504,067 states that the marigold oleoresin is pre treated with Sodium Carbonate and further neutralization with dilute Phosphoric acid, prior to taking it to saponification reaction using aqueous alkali and carried out the saponification at a temperature at 90°C for 8 hours. Subsequently the reaction mass is subjected to readjustment of pH with acetic acid to 5.0, and washing the residues with excess water in order to bring the pH to neutral. The disadvantage in the process is that the product is subjected to heat for a prolonged period and too many steps of acidification and neutralizations are involved to remove the impurities.

### BRIEF SUMMARY OF THE INVENTION

A first aspect of the present invention relates to a process for isolation of carotenoid crystals having lutein and zeaxanthin in a weight ratio of 10:1, 5:1 or 1:1. The process comprises contacting a plant source rich in lutein with hexane and extracting at a temperature of 40°C to 60°C to obtain an oleoresin rich in lutein; contacting a plant source rich in zeaxanthin with hexane and extracting at a temperature of 40°C to 60°C to obtain an oleoresin rich in zeaxanthin. The oleoresin rich in lutein and the oleoresin rich in zeaxanthin are mixed separately in a ratio ranging from 80:20(w/w) to 90:10(w/w) or 70:30(w/w) to 30:70(w/w) or 10:90(w/w) to 20:80(w/w) and homogenized to obtain a mixed oleoresin. The mixed oleoresin is hydrolyzed with an alcoholic alkali at a temperature of 70°C to 80°C to obtain a reaction mixture. The carotenoid crystals are precipitated by adding hot water to the reaction mixture to form a precipitate. Carotenoid crystals having lutein and zeaxanthin in a weight ratio of 10:1 1 or 5:1 or 1:1, respectively, are obtained by filtering, washing and drying the precipitate.

The plant source rich in lutein and the plant source rich in zeaxanthin may be independently selected from the group consisting of marigold flowers (*Tagetes erecta*)*,* marigold petals, mutant marigold flowers, mutated marigold petals, fruits of paprika, and berries of Chinese wolfberries (*Lycium barbarum*)*.* In a specific embodiment, the plant source rich in lutein is selected from the group consisting of marigold flowers and marigold petals, and the plant source rich in zeaxanthin is selected from the group consisting of mutant marigold flowers, mutated marigold petals, fruits of paprika, and berries of Chinese wolfberries.

The process of the present invention enables a rapid and efficient extraction of the carotenoids from the plant sources employed. Since the plant material is not subj ected to heat for a longer duration, the formation of adverse side products (i.e. degenerated oxidative products) is eliminated or at least reduced. Typically, the whole process can be completed within 3 hours. The process of the invention is characterized by the use of rather mild temperature conditions and of rather low amounts of solvents as well as the overall convenience of the procedure. All these factors contribute towards the yield and stability of the carotenoid crystals obtained and bring down the cost of production on a commercial scale.

Another aspect of the present invention relates to carotenoid crystals comprising lutein and zeaxanthin *per se*, wherein a weight ratio of lutein to zeaxanthin is 10:1, 5:1 or 1:1 are also aspects of the present disclosure. Carotenoid crystals with these weight ratios made by the process as described above are also part of this invention. These ratios of lutein to zeaxanthin, specifically 10:1, 5:1 1 or 1:1, are critical in making the carotenoids more bioactive. The carotenoid crystals comprising lutein and zeaxanthin, wherein a weight ratio of lutein to zeaxanthin is 10:1, 5:1 or 1:1 are useful as antioxidants. These carotenoid crystals are particularly good for eye care.

Finally, a third aspect of the present invention relates to the use of carotenoid crystals comprising a weight ratio of lutein to zeaxanthin of 10:1 or 5:1 or 1:1 as antioxidants.

### DETAILED DESCRIPTION OF THE INVENTION

The present invention will be described with respect to particular embodiments but the invention is not limited thereto but only by the claims.

Where the term "comprising" is used in the present description and claims, it does not exclude other elements or steps. For the purposes of the present invention, the term "consisting of" is considered to be a preferred embodiment of the term "comprising of'. If hereinafter a group is defined to comprise at least a certain number of embodiments, this is also to be understood to disclose a group, which preferably consists only of these embodiments.

Where an indefinite or definite article is used when referring to a singular noun e.g. "a" or "an", "the", this includes a plural of that noun unless something else is specifically stated.

Where numerical values are given in the context of the present invention, the person skilled in the art will understand that the technical effect of the feature in question will be ensured within an interval of accuracy. The term "interval of accuracy" typically indicates a deviation from the indicated numerical value of ± 10%, and preferably of ± 5%.

Conventional methods for preparing the carotenoid crystals having both lutein and zeaxanthin, such as crystal blending after purifying lutein crystals and zeaxanthin crystals individually, results in varied ratios of lutein and zeaxanthin. Further, with such methods the recoveries of zeaxanthin purified crystals obtained is very low which is not economical on a commercial scale. Thus, there is also a need for a process for isolation of carotenoid crystals rich in lutein and zeaxanthin from plant sources, to obtain carotenoid crystals of lutein and zeaxanthin in the weight ratios of 10:1, 5:1 and 1:1.

In one aspect, the present invention provides carotenoid crystals comprising lutein and zeaxanthin in weight ratios of 10:1, 5:1, and 1:1, respectively. The present invention also relates to a corresponding process for the isolation of carotenoid crystals having lutein and zeaxanthin in weight ratios of 10:1, 5:1, and 1:1, respectively. The process comprises contacting a plant source rich in lutein with hexane and extracting at a temperature in the range of 40°C to 60°C to obtain an oleoresin rich in lutein, and contacting plant source rich in zeaxanthin with hexane and extracting at a temperature in the range of 40° C to 60°C to obtain an oleoresin rich in zeaxanthin. The oleoresin rich in lutein is then mixed with the oleoresin rich in zeaxanthin in ratios ranging from 80:20(w/w) to 90:10(w/w), or 70:30(w/w) to 30:70(w/w) or 10:90(w/w) to 20:80(w/w), respectively. The mixed oleoresin is hydrolyzed with an alcoholic alkali at a temperature in the range of 70°C to 80°C to obtain a reaction mixture. Subsequently, the carotenoid crystals are precipitated by adding hot water to the reaction mixture, wherein ratio of reaction mixture to the hot water is preferably in the range of 1:1 to 1:1.5 (v/v). The carotenoid crystals having lutein and zeaxanthin in a weight ratio of 10:1, 5:1 and 1:1, respectively, are obtained by filtering, washing, and drying the precipitate.

In a specific embodiment, the plant source rich in lutein and the plant source rich in zeaxanthin may be independently selected from the group consisting of marigold flowers (*Tagetes erecta*)*,* marigold petals, mutant marigold flowers, mutated marigold petals, fruits of paprika, and berries of Chinese wolfberries (*Lycium barbarum*)*.*

Typically, the plant material employed is harvested and then immediately taken for silaging in silos after physical cleaning and sprayed with anti oxidant and silage additive at appropriate concentration under closed anaerobic conditions. The silaging is monitored through pH and temperature of the silage and ensured for complete fermentation over a period of usually two to three weeks.

The silaged plant material is then usually harvested from the silos and subjected to a dehydration process in series of steps. The silaged plant material is subjected to an industrial screw press in two stages and squeezed for the oozing water, bringing the moisture content from 88% to 75%. The squeezed plant material is then subjected to shredding before it is dried in fluid bed drier. The shredded flowers are dried in a fluid bed drier using hot air, generated by heating air with producer-gas flame produced by using an eco-friendly gasifier with absolutely stack free emission. The tunnel type industrial fluid bed drier (FBD) comprises of drying chambers with different temperatures across the tunnel from inlet, being the maximum temperature (85°C to 90°C) to the outlet at temperature (45°C to 50°C).

Typically, the transit time inside the FBD from inlet to outlet is only up to 30 minutes, wherein the moisture level in the product is brought down to around 10% from 75%. The advantage in this drying process is that the product is not subjected to high heat for longer duration, minimizing the formation of degenerative oxidative products that could form due to heat and air for prolonged periods.

Typically, the dried plant material is pulverized using an industrial hammer mill and down sized to particles less than 400 microns. The ground material is then pelletized to 6mm to 10mm size pellets using an industrial pelletizer to the desired bulk density with the aid of steam/hot water as binder.

Subsequently, the pellets are subjected to solvent extraction using food grade hexanes as solvent in a battery of extractors under counter current extraction to achieve maximum extractability of active principles viz., xanthophylls and carotenoids along with the other resinoids and lipids. The lean miscella is then concentrated in falling film evaporators and wiped film evaporators to bring down the solvent concentration to around 5% from 90% to 95% approximately. The concentrated miscella is then subjected to vacuum distillation to bring down the solvent level from 5% to 1%, further concentrated by stripping the solvent under a stream of nitrogen and steam to reduce the solvent levels to less than 1000 ppm in the final oleoresin. Throughout the concentration operation, the product is not subjected to temperatures more than 60°C at any given point of time, minimizing the formation of oxidative degenerated products like epoxides.

Within the present invention, the plant source rich in lutein that is used for obtaining an oleoresin rich in lutein) and the plant source rich in zeaxanthin that is used for obtaining an oleoresin rich in zeaxanthin are harvested and extracted separately, resulting in two types of oleoresins. Then, the oleoresin rich in lutein and the oleoresin rich in zeaxanthin are mixed separately in a ratio ranging from 80:20(w/w) to 90:10(w/w) or 70:30(w/w) to 30:70(w/w) or 10:90(w/w) to 20:80(w/w) and homogenized to obtain a mixed oleoresin.

In some embodiments of the invention, a ratio of the oleoresin rich in lutein to the oleoresin rich in zeaxanthin ranging from 80:20(w/w) to 90:10(w/w) is employed for isolating carotenoid crystals having lutein and zeaxanthin in a weight ratio of 10:1.

In other embodiments of the invention, a ratio of the oleoresin rich in lutein to the oleoresin rich in zeaxanthin ranging from 70:30(w/w) to 30:70(w/w) is employed for isolating carotenoid crystals having lutein and zeaxanthin in a weight ratio of 5:1.

In still other embodiment of the invention, a ratio of the oleoresin rich in lutein to the oleoresin rich in zeaxanthin ranging from 10:90(w/w) to 20:80 is employed for isolating carotenoid crystals having lutein and zeaxanthin in a weight ratio of 1:1.

After mixing, the (combined) oleoresin obtained is usually homogenized in a reactor under stirring at a temperature not exceeding 45°C for a period of maximum 10 minutes.

The homogenized oleoresin is then hydrolyzed in the same reactor by adding an alcoholic alkali. In specific embodiments, the alcoholic alkali is selected from the group consisting of ethanolic sodium hydroxide and ethanolic potassium hydroxide. In other specific embodiments, the concentration of the alcoholic alkali is 10%-30%.

Typically, 1.2 to 2.0 volumes of 13% to 15% alcoholic alkali solution, of the quantity of the mixed oleoresin, are added. Incubation is typically performed at a temperature ranging between 70°C and 80°C for a time period of not more than 30 minutes, wherein the alcohol used is absolute ethyl alcohol with moisture content less than 5%. The degree of saponification is ensured by either thin layer chromatography or high pressure liquid chromatography and the final cooking is done for 10 minutes at the same temperature after ensuring the completion of saponification more than 99%.

To the saponified mass, hot water generated in a separate vessel at a temperature of usually 65°C to 75°C is added and homogenized well for about 10 minutes at the same temperature to aid the crystallization of carotenoids. In specific embodiments, the ratio of the reaction mixture to hot water is in the range of about 1:1 to 1:1.5 (v/v).

The diluted mass is then typically filtered through a filter press by pumping the mass into the filter press aided by positive pressure using either nitrogen or air. The collected mass inside the filter press plate is given with a hot water wash at a temperature of 55°C to 80° C with sufficient quantity of hot water until the pH is brought down to neutral at around 7.0.

The wet mass collected from the filter press is taken in trays in thin layers and dried in tray drier at a temperature between 50°C and 55°C at atmospheric pressure or in a vacuum tray drier at reduced pressure at a temperature between 40°C and 45°C for the time (usually 3 to 4 hours) until the moisture level in the product is less than 1% and any hazardous organic volatile impurity is below the detectable limit determined by gas chromatography.

The carotenoid crystals obtained are usually formulated and stabilized in bulk, in the form of powder, beadlets, granules, oil dispersions and water dispersions with concentrations varying from 1% to 40% concentrations by adding suitable pharma grade excipients and emulsifiers depending upon the end usage in line with the nutraceutical and food products applications.

In an embodiment, the present disclosure provides carotenoid crystals comprising lutein and zeaxanthin, wherein the weight ratio of the lutein to zeaxanthin is 10:1.

In another embodiment, the present disclosure provides carotenoids crystals comprising lutein and zeaxanthin, wherein the weight ratio of the lutein to zeaxanthin is 5:1.

In another embodiment, the present disclosure provides carotenoids crystals comprising lutein and zeaxanthin, wherein the weight ratio of the lutein to zeaxanthin is 1:1.

Yet another embodiment of the present disclosure provides a process for isolation of carotenoid crystals having lutein and zeaxanthin in a weight ratio of 10:1, said process comprising; contacting a plant source rich in lutein with hexane and extracting at a temperature of 40°C to 60°C to obtain an oleoresin rich in lutein; contacting a plant source rich in zeaxanthin with hexane and extracting at a temperature of 40°C to 60°C to obtain an oleoresin rich in zeaxanthin; mixing the oleoresin rich in lutein and the oleoresin rich in zeaxanthin in a ratio ranging from 80:20 (w/w) to 90:10 (w/w) and homogenizing to obtain a mixed oleoresin; hydrolyzing the mixed oleoresin with an alcoholic alkali at a temperature of 70°C to 80°C to obtain a reaction mixture; precipitating carotenoid crystals by adding hot water to the reaction mixture to form a precipitate; and obtaining carotenoid crystals having lutein and zeaxanthin in a weight ratio of 10:1. The carotenoid crystals having lutein and zeaxanthin in a ratio of 10:1 1 are obtained by filtering, washing and drying the precipitate.

Yet another embodiment of the present disclosure provides a process for isolation of carotenoid crystals having lutein and zeaxanthin in a weight ratio of 10:1, wherein the plant source rich in lutein and the plant source rich in zeaxanthin are independently selected from the group consisting of marigold flowers (*Tagetes erecta*)*,* marigold petals, mutant marigold flowers, mutant marigold petals, fruits of paprika and berries of Chinese wolfberries (*Lycium barbarum*)*.* In a particular embodiment, the plant source rich in lutein is selected from the group consisting of marigold flowers and marigold petals, and the plant source rich in zeaxanthin is selected from the group consisting of mutant marigold flowers, mutated marigold petals, fruits of paprika, and berries of Chinese wolfberries.

Yet another embodiment of the present disclosure provides a process for isolation of carotenoid crystals having lutein and zeaxanthin in a weight ratio of 10:1, wherein the alcoholic alkali is selected from the group consisting of ethanolic sodium hydroxide and ethanolic potassium hydroxide.

Yet another embodiment of the present disclosure provides a process for isolation of carotenoid crystals having lutein and zeaxanthin in a weight ratio of 10:1, wherein the alcoholic alkali is 10%-30%.

Yet another embodiment of the present disclosure provides a process for isolation of carotenoid crystals having lutein and zeaxanthin in a weight ratio of 10:1, wherein the alcoholic alkali is 10%-30% ethanolic potassium hydroxide.

Yet another embodiment of the present disclosure provides a process for isolation of carotenoid crystals having lutein and zeaxanthin in a weight ratio of 10:1, wherein the ratio of the reaction mixture to the hot water is in the range of about 1:1 to 1:1.5(v/v).

Yet another embodiment of the present disclosure provides a process for isolation of carotenoid crystals having lutein and zeaxanthin in a weight ratio of 10:1, wherein the washing is carried out with hot water at a temperature in the range of 55°C to 80°C.

Yet another embodiment of the present disclosure provides a process for isolation of carotenoid crystals having lutein and zeaxanthin in a weight ratio of 10:1, wherein the washing is carried out at 75°C.

Yet another embodiment of the present disclosure provides a process for isolation of carotenoid crystals having lutein and zeaxanthin in a weight ratio of 10:1, wherein the drying of carotenoids crystals is carried out at a temperature in the range of 55°C to 60°C.

Yet another embodiment of the present disclosure provides a process for isolation of carotenoid crystals having lutein and zeaxanthin in a ratio of 5:1, said process comprising: contacting a plant source rich in lutein with hexane and extracting at a temperature of 40°C to 60°C to obtain an oleoresin rich in lutein; contacting a plant source rich in zeaxanthin with hexane and extracting at a temperature of 40°C to 60°C to obtain an oleoresin rich in zeaxanthin; mixing the oleoresin rich in lutein and the oleoresin rich in zeaxanthin in a ratio ranging from 70:30(w/w) to 30:70(w/w) and homogenizing to obtain a mixed oleoresin; hydrolyzing the mixed oleoresin with an alcoholic alkali at a temperature of 70°C to 80°C to obtain a reaction mixture; precipitating carotenoid crystals by adding hot water to the reaction mixture to form a precipitate; and (1: 4 volumes is the ratio of the oleoresin to water which corresponds to about 1:1.4 volumes calculated to ratio of the reaction mixture to water) obtaining carotenoid crystals having lutein and zeaxanthin in a ratio of 5:1. The carotenoid crystals having lutein and zeaxanthin in a ratio of 5:1 are obtained by filtering, washing and drying the precipitate.

Yet another embodiment of the present disclosure provides a process for isolation of carotenoid crystals having lutein and zeaxanthin in a weight ratio of 5:1, wherein the plant source rich in lutein and the plant source rich in zeaxanthin are independently selected from the group consisting of marigold flowers (*Tagetes erecta*)*,* marigold petals, mutant marigold flowers, mutant marigold petals, fruits of paprika and berries of Chinese wolfberries (*Lycium barbarum*)*.* In a particular embodiment, the plant source rich in lutein is selected from the group consisting of marigold flowers and marigold petals, and the plant source rich in zeaxanthin is selected from the group consisting of mutant marigold flowers, mutated marigold petals, fruits of paprika, and berries of . Chinese wolfberries.

Yet another embodiment of the present disclosure provides a process for isolation of carotenoid crystals having lutein and zeaxanthin in a weight ratio of 5:1, wherein the alcoholic alkali is selected from the group consisting of ethanolic sodium hydroxide and ethanolic potassium hydroxide.

Yet another embodiment of the present disclosure provides a process for isolation of carotenoids crystals having lutein and zeaxanthin in a weight ratio of 5:1, wherein the alcoholic alkali is 10%-30%.

Yet another embodiment of the present disclosure provides a process for isolation of carotenoids crystals having lutein and zeaxanthin in a weight ratio of 5:1, wherein the alcoholic alkali is 10%-30% ethanolic potassium hydroxide.

Yet another embodiment of the present disclosure provides a process for isolation of carotenoid crystals having lutein and zeaxanthin in a weight ratio of 5:1, wherein ratio of the reaction mixture to the hot water is in the range of 1:1 to 1:1.5 (v/v). Yet another embodiment of the present disclosure provides a process for isolation of carotenoid crystals having lutein and zeaxanthin in a weight ratio of 5:1, wherein the washing is carried out with hot water at a temperature in the range of 55°C to 80°C.

Yet another embodiment of the present disclosure provides a process for isolation of carotenoids crystals having lutein and zeaxanthin in a weight ratio of 5:1, wherein the washing is carried out at 75°C.

Yet another embodiment of the present disclosure provides a process for isolation of carotenoids crystals having lutein and zeaxanthin in a weight ratio of 5:1, wherein the drying of carotenoids crystals is carried out at a temperature in the range of 55°C to 60°C.

Yet another embodiment of the present disclosure provides a process for isolation of carotenoids crystals having lutein and zeaxanthin in a ratio of 1:1, said process comprising: contacting a plant source rich in lutein with hexane and extracting at a temperature of 40°C to 60°C to obtain an oleoresin rich in lutein; contacting a plant source rich in zeaxanthin with hexane and extracting at a temperature of 40°C to 60°C to obtain an oleoresin rich in zeaxanthin; mixing the oleoresin rich in lutein and the oleoresin rich in zeaxanthin in a ratio ranging from 10:90(w/w) to 20:80(w/w) and homogenizing to obtain a mixed oleoresin; hydrolyzing the mixed oleoresin with an alcoholic alkali at a temperature of 70°C to 80°C to obtain a reaction mixture; precipitating carotenoid crystals by adding hot water to the reaction mixture to form a precipitate; and obtaining carotenoid crystals having lutein and zeaxanthin in a ratio of 1:1. The carotenoid crystals having lutein and zeaxanthin in a ratio of 1:1 are obtained by filtering, washing and drying the precipitate.

Yet another embodiment of the present disclosure provides a process for isolation of carotenoid crystals having lutein and zeaxanthin in a ratio of 1:1, wherein the plant source rich in lutein and the plant source rich in zeaxanthin are independently selected from the group consisting of marigold flowers (*Tagetes erecta*), marigold petals, mutant marigold flowers, mutant marigold petals, fruits of paprika, and berries of Chinese wolfberries (*Lycium barbarum*). In a particular embodiment, the plant source rich in lutein is selected from the group consisting of marigold flowers and marigold petals, and the plant source rich in zeaxanthin is selected from the group consisting of mutant marigold flowers, mutated marigold petals, fruits of paprika, and berries of Chinese wolfberries.

Yet another embodiment of the present disclosure provides a process for isolation of carotenoid crystals having lutein and zeaxanthin in a ratio of 1:1, wherein the alcoholic alkali is selected from the group consisting of ethanolic sodium hydroxide and ethanolic potassium hydroxide.

Yet another embodiment of the present disclosure provides a process for isolation of carotenoid crystals having lutein and zeaxanthin in a ratio of 1:1, wherein the alcoholic alkali is 10%-30%.

Yet another embodiment of the present disclosure provides a process for isolation of carotenoid crystals having lutein and zeaxanthin in a ratio of 1:1, wherein the alcoholic alkali is 10%-30% ethanolic potassium hydroxide.

Yet another embodiment of the present disclosure provides a process for isolation of carotenoid crystals having lutein and zeaxanthin in a ratio of 1:1, wherein ratio of reaction mixture to the hot water is in the range of 1:1 to 1:1.5 (v/v).

Yet another embodiment of the present disclosure provides a process for isolation of carotenoid crystals having lutein and zeaxanthin in a ratio of 1:1, wherein the washing is carried out with hot water at a temperature in the range of 55°C to 80°C.

Yet another embodiment of the present disclosure provides a process for isolation of carotenoid crystals having lutein and zeaxanthin in a ratio of 1:1 wherein the washing is carried out at 75°C.

Yet another embodiment of the present disclosure provides a process for isolation of carotenoid crystals having lutein and zeaxanthin in a ratio of 1:1, wherein the drying of carotenoid crystals is carried out at a temperature in the range of 55°C to 60°C.

Yet another embodiment of the present disclosure provides carotenoid crystals having lutein and zeaxanthin in a weight ratio of 10:1, made by a process comprising: contacting a plant source rich in lutein with hexane and extracting at a temperature of 40°C to 60°C to obtain an oleoresin rich in lutein; contacting a plant source rich in zeaxanthin with hexane and extracting at a temperature of 40°C to 60°C to obtain an oleoresin rich in zeaxanthin; mixing the oleoresin rich in lutein and the oleoresin rich in zeaxanthin in a ratio ranging from 80:20(w/w) to 90:10(w/w) and homogenizing to obtain a mixed oleoresin; hydrolyzing the mixed oleoresin with an alcoholic alkali at a temperature of 70°C to 80°C to obtain a reaction mixture; precipitating carotenoid crystals by adding hot water to the reaction mixture to form a precipitate; and obtaining carotenoid crystals having lutein and zeaxanthin in a weight ratio of 10:1. The carotenoid crystals having lutein and zeaxanthin in a ratio of 10: 1 are obtained by filtering, washing and drying the precipitate.

Yet another embodiment of the present disclosure provides carotenoid crystals having lutein and zeaxanthin in a weight ratio of 5:1, made by a process comprising: contacting a plant source rich in lutein with hexane and extracting at a temperature of 40°C to 60°C to obtain an oleoresin rich in lutein; contacting a plant source rich in zeaxanthin with hexane and extracting at a temperature of 40°C to 60°C to obtain an oleoresin rich in zeaxanthin; mixing the oleoresin rich in lutein and the oleoresin rich in zeaxanthin in a ratio ranging from 70:30(w/w) to 30:70(w/w) and homogenizing to obtain a mixed oleoresin; hydrolyzing the mixed oleoresin with an alcoholic alkali at a temperature of 70°C to 80°C to obtain a reaction mixture; precipitating carotenoid crystals by adding hot water to the reaction mixture to form a precipitate; and obtaining carotenoid crystals having lutein and zeaxanthin in a ratio of 5:1. The carotenoid crystals having lutein and zeaxanthin in a ratio of 5:1 are obtained by filtering, washing and drying the precipitate.

Yet another embodiment of the present disclosure provides carotenoid crystals having lutein and zeaxanthin in a weight ratio of 1:1, made by a process comprising: contacting a plant source rich in lutein with hexane and extracting at a temperature of 40°C to 60°C to obtain an oleoresin rich in lutein; contacting a plant source rich in zeaxanthin with hexane and extracting at a temperature of 40°C to 60°C to obtain an oleoresin rich in zeaxanthin; mixing the oleoresin rich in lutein and the oleoresin rich in zeaxanthin in a ratio ranging from 10:90(w/w) to 20:80(w/w) and homogenizing to obtain a mixed oleoresin; hydrolyzing the mixed oleoresin with an alcoholic alkali at a temperature of 70°C to 80°C to obtain a reaction mixture; precipitating carotenoids crystals by adding hot water to the reaction mixture to form a precipitate; and obtaining carotenoids crystals having lutein and zeaxanthin in a ratio of 1:1. The carotenoids crystals having lutein and zeaxanthin in a ratio of 1:1 are obtained by filtering, washing, and drying the precipitate.

Another embodiment of the present invention provides use of carotenoid crystals comprising a weight ratio of the lutein to zeaxanthin in 10:1 1 or 5:1 or 1:1 as antioxidants.

While the invention has been described with reference to the explained embodiment, it is not limiting to anybody's skill to make various changes or equivalents without altering or departing from the main scope of this invention. Therefore, it is intended that the invention not be limiting to the embodiment described but will cover and include all other embodiments falling within the scope of the claims made herein.

The following examples are illustrative, but not limiting, of the methods and compositions of the present invention. Other suitable modifications and adaptations of the variables of the conditions and normally encountered in natural products isolation and purification techniques are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

### EXAMPLES

### EXAMPLE 1

25 kg of marigold oleoresin having 92.19gm/Kg or 9.22% of Xanthophylls is taken in 100 liters capacity reactor with an agitator. The Oleoresin is homogenized for 10 minutes under stirring at a temperature of about 40°C with either steam or hot water in the jacket of the reactor as heating medium. Ethanolic KOH is prepared by taking 5 kg of KOH with purity of 95% and dissolving it in 35 liters of Ethyl alcohol (1:1.4 volumes). The prepared ethanolic KOH is added into reaction vessel slowly, containing the homogenized mass. The saponification reaction is carried out at a temperature of about 75°C for 30 minutes. After ensuring the degree of saponification to be more than 99% by HPLC, 40 liters of demineralized hot water maintained at a temperature of 70°C is added to the reacted mass and the stirring is continued for 10 minutes. The diluted mass with carotenoid crystals is then pumped into a filter press to recover the crystals.

Around 250 liters of additional hot water is pumped through the filter press to wash the unwanted impurities and bring down the pH of the effluent to neutral around 7.0. After ensuring the neutralization, positive pressure of Nitrogen is applied to the filter press at pressure 1.25 kg to squeeze the crystals trapped inside the filter. The wet crystals are then collected from the filter press plates into trays in a thin layer and dried in a tray drier at a temperature around 55°C for 3 hours under normal pressure.

The physical recovery of the final product is 6.76%. The carotenoid crystals obtained contained 91.28% carotenoids (determined by spectrophotometer) of which 91.99% is all trans-lutein, 6.90% all trans-zeaxanthin, 0.27% cis-luteins, 0.23% beta-carotene and 0.5% cryptoxanthin (determined by HPLC). The chemical recovery of the final product is 66.9%.

The final product contained a moisture content of 0.57% and could not be detected for any traces of residual hexanes by gas chromatography analysis.

### EXAMPLE 2

25.5 kg of marigold oleoresin having 102.18gm/kg or 10.22% of xanthophylls is taken in 100 liters capacity reactor with an agitator.

The oleoresin is homogenized for 10 minutes under stirring at a temperature of about 45°C with either steam or hot water in the jacket of the reactor as heating medium. Ethanolic KOH is prepared by taking 5.1 kg of KOH with purity of 95% and dissolving it 40 liters of ethyl alcohol (1:1.56 volumes).

The prepared ethanolic KOH is added into reaction vessel slowly, containing the homogenized mass. The saponification reaction is carried out at a temperature of 73°C for 30 minutes. After ensuring the degree of saponification to be more than 99% by HPLC, 45 liters of demineralized hot water maintained at a temperature of about 65°C is added to the reacted mass and the stirring is continued for 10 minutes. The diluted mass with carotenoid crystals is then pumped into a filter press to recover the crystals. Around 275 liters of additional hot water is pumped through the filter press to wash the unwanted impurities and bring down the pH of the effluent to neutral around 7.0. After ensuring the neutralization, positive pressure of Nitrogen is applied to the filter press at pressure 1.2 Kg to squeeze the crystals trapped inside the filter. The wet crystals are then collected from the filter press plates into trays in a thin layer and dried in a tray drier at a temperature around 45°C for 2 hours under vacuum at 600 mm Hg.

The physical recovery of the final product is 7.67%. The carotenoid crystals obtained contained 93.76% carotenoids (determined by spectrophotometer) of which 92.86% is all trans-lutein, 6.14% all trans-zeaxanthin, 0.12% cis-lutein, 0.22% beta-carotene and 0.52% cryptoxanthin (determined by HPLC). The chemical recovery of the final product is 70.53%.

The final product contained a moisture content of 0.63% and could not be detected for any traces of residual hexanes by gas chromatography analysis.

### EXAMPLE 3

20 kg of lutein rich marigold meal having 8.99 gm/kg (0.89%) of total carotenoids (with a carotenoids profile of 74.58% trans-lutein (T-lutein), 4.28% trans-zeaxanthin, 1.94% of beta-carotene, 1.02% of cryptoxanthin and 18.08% cis-isomers and epoxides measured by HPLC) was taken in a 200 liters capacity extractor with circulation facility. 120 liters of food grade hexane with a gas chromatographic purity of more than 98%, was added to the extractor and circulated. The temperature was raised to about 55°C and maintained for 1 hour, under circulation. After 1 hour the extract was drained and collected in a miscella tank. The extraction was repeated for two more times each with 120 liters of Hexane under the same conditions of temperature and time. The extracts obtained were collected in the miscella tank. The fourth and fifth extractions were carried out using 100 liters of hexane in each extraction under circulation and at a temperature of 58°C. 512 liters of miscella collected from the five extractions was taken to an evaporator, preferably a falling film evaporator or a wiped film evaporator to obtain a concentrated miscella containing 70% solids and 30% solvent. The concentrated miscella was distilled further in a distillation unit under atmospheric pressure to increase the concentration of solids to 95% with a solvent level of around 5%. The solvent was distilled further under reduced pressure (450 to 600 mm Hg) to reduce the solvent level further to less than 1%. The total amount of solvent recovered was 472 liters with a gas chromatographic purity of more than 98%. 1.624 kg of oleoresin containing 105.3 g/kg of carotenoids (10.53% carotenoids) was obtained with a carotenoids chemical recovery of 95.11%. The oleoresin thus obtained exhibited a carotenoids profile containing 74.82% trans-lutein, 5.43% T-zeaxanthin, 1.56% of beta-carotene, 0.80% of cryptoxanthin and 17.38% of cis-isomers /epoxides measured by HPLC.

### EXAMPLE 4

20 kg of zeaxanthin rich marigold meal (obtained from Ball Horticulture Inc covered by the U.S. Patent No. 6,784,351) having 3.29 g/kg (0.329%) of total carotenoids with a carotenoid profile of 59.08% T-zeaxanthin, 14.80% of beta-carotene, 11.77% ofT-lutein, 2.76% of alpha-cryptoxanthin, 2.33% of beta-cryptoxanthin, 1.98% of alpha-carotene, 0.82% of cis-lutein, 0.22% of chrysanthemaxanthin and 6.24% of other unidentified carotenoids, measured by HPLC was taken in a 200 liters capacity extractor with circulation facility. 120 liters of Food grade hexane with a Gas chromatographic purity of more than 98%, was added to the extractor and circulated. The temperature was raised to about 45°C and maintained for 1 hour under circulation. After 1 hour, the extract was drained and collected in a miscella tank. The extraction was repeated for 3 more times with 60 liters of Hexane under the same conditions of temperature and time. The extracts obtained were collected in the miscella tank. 255 liters of miscella collected from the above 4 extractions was taken to an evaporator, preferably a falling film evaporator or a wiped film evaporator to obtain a concentrated miscella containing 70% solids and 30% solvent. The concentrated miscella was distilled further in a distillation unit under atmospheric pressure to increase the concentration of solids to 95% with a solvent level of around 5%. The solvent was distilled further under reduced pressure (450 to 600 mm Hg) to reduce the solvent level further to less than 1%. The total amount of solvent recovered was 218.8 liters with gas chromatographic purity of more than 98%. 1.592 kg of oleoresin containing 41.26 g/kg of total carotenoids (4.126% of total carotenoids) was obtained with a recovery of 99.82%. The zeaxanthin chemical recovery was 92.07%. The oleoresin thus obtained exhibited a carotenoids profile containing 54.49% of T-zeaxanthin, 9.83% of beta-carotene, 22.74% of T-lutein, 3.07% of alpha-cryptoxanthin, 1.93% ofbeta-cryptoxanthin, 2.75% of alpha-carotene, 0% of cis-lutein and 0.07% of chrysanthemaxanthin and 5.12% of other unidentified carotenoids, measured by HPLC.

### EXAMPLE 5

The lutein rich marigold oleoresin obtained from Example 3 and the zeaxanthin rich marigold oleoresin obtained from Example 4 are blended in the ratio of 86:14 by physical weight, respectively. 0.45 kg of blended marigold oleoresin containing 96.28 g/kg of carotenoids (9.63% carotenoids) was homogenized for around 10 minutes in a 5.0 liters capacity 3 necked round bottom flasks under continuous stirring at a temperature of about 40°C in a hot water bath. Alcoholic KOH was prepared by taking 90 g of KOH (20% calculated to the input Oleoresin) with the purity of around 90% and dissolving it in 675 ml (1:1.5 volumes to the Oleoresin) of ethanol. The prepared alcoholic KOH was slowly added into reaction vessel containing the homogenized marigold oleoresin. The saponification reaction was carried out at a temperature of about 75°C for 30 minutes. After ensuring the degree of saponification to be more than 99% by HPLC, 1800 ml of demineralized hot water (1: 4 volumes is the ratio of the oleoresin to water which corresponds to about 1:1.4 volumes calculated to ratio of the reaction mixture to water) maintained at a temperature of about 75°C was added to the saponified marigold oleoresin and stirring was continued for about 10 minutes. The diluted saponified m arigold oleoresin comprising carotenoid crystals was filtered in a buchner funnel to recover the carotenoid crystals. The carotenoid crystals thus obtained were washed with 3600 ml (1:8 volumes calculated to the oleoresin) of hot water maintained at around 75°C to remove the impurities and bring down the pH of the effluent to neutral around 7.0. The wet crystals were then collected from the filter and dried in a Fluid bed drier at a temperature of around 55°C for 1 hour or until the moisture level is brought to less than 0.3% and the hexane and alcohol levels are less than 10 ppm and 100 ppm, respectively.

36.81 g of purified carotenoids crystals rich in T-lutein and T-zeaxanthin were obtained and the physical recovery of the final product was 8.18%. The carotenoid crystals thus obtained contained 91.39% total carotenoids determined by UV-Visible spectrophotometer of which 89.84% was all T-lutein, 9.04% all T-zeaxanthin, 0.43 % beta-cryptoxanthin, and 0.37% of beta-carotene and without any traces of cis-luteins and epoxides, determined by HPLC.

The carotenoids crystals thus obtained contain 82.10% of T-lutein by weight and 8.26% by weight of T-zeaxanthin with the ratio of T-lutein to T-zeaxanthin by weight to be at about 10:1 ratio (9.94).

The carotenoid crystals thus obtained contained 8.06% of wax content when measured using gas chromatography. The carotenoids chemical recovery of the final product was 77.63% with a lutein chemical recovery of 97.2% and a zeaxanthin chemical recovery of 92.4%. The final product contained a moisture content of 0.29% with 68.78 ppm of ethanol and 2.36 ppm of hexanes detected by gas chromatography.

### EXAMPLE 6

The lutein rich marigold oleoresin obtained in Example 3 and the zeaxanthin rich marigold oleoresin obtained in Example 4 are blended in the ratio of 50:50 by physical weight, respectively. 0.45 kg of blended marigold oleoresin containing 73.88 g/kg of carotenoids (7.388% carotenoids) was homogenized for 10 minutes in a 5.0 liters capacity 3 necked round bottom flasks under continuous stirring at a temperature of 40° C in a hot water bath. Alcoholic KOH was prepared by taking 90 g of KOH (20% calculated to the input oleoresin) with the purity of around 90% and dissolving it in 675 ml (1:1.5 volumes to the oleoresin) of ethanol. The prepared alcoholic KOH was slowly added into reaction vessel containing the homogenized marigold oleoresin. The saponification reaction was carried out at a temperature of about 75° C for 30 minutes. After ensuring the degree of saponification to be more than 99% by HPLC, 1800 ml of demineralized hot water (1: 4 volumes is the ratio of the oleoresin to water which corresponds to about 1:1.4 volumes calculated to ratio of the reaction mixture to water) maintained at a temperature of about 75°C was added to the saponified marigold oleoresin and stirring was continued for 10 minutes. The diluted saponified marigold oleoresin comprising carotenoid crystals was filtered in a buchner funnel to recover the carotenoid crystals. The carotenoid crystals thus obtained were washed with 3600 ml (1:8 volumes calculated to the oleoresin) of hot water maintained at around 75°C to remove the impurities and bring down the pH of the effluent to neutral around 7.0. The wet crystals were then collected from the filter and dried in a fluid bed drier at a temperature of around 55°C for 1 hour or until the moisture level is brought to less than 0.3 % and the hexane and alcohol levels are less than 10 ppm and 100 ppm, respectively.

27.45g of purified carotenoids crystals rich in T-lutein and T-zeaxanthin were obtained and the physical recovery of the final product was 6.1%. The carotenoid crystals thus obtained contained 90.71% total carotenoids determined by UV-Visible spectrophotometer of which 81.80% was all T-lutein, 16.2% all T-zeaxanthin, 0.69% beta-cryptoxanthin, and 0.95% of beta-carotene and without any traces of cis-luteins and epoxides, determined by HPLC.

The carotenoids crystals thus obtained contain 74.2% of T-lutein by weight and 14.69% by weight of T-zeaxanthin with the ratio of T-lutein to T-zeaxanthin by weight to be at about 5:1 ratio (5.05).

The carotenoid crystals thus obtained contained 9.20% of wax content when measured using gas chromatography. The carotenoids chemical recovery of the final product was 74.89% with a lutein chemical recovery of 79.99% and a zeaxanthin chemical recovery of 83.20%. The final product contained a moisture content of 0.30% with 40.30 ppm of ethanol and 6.86 ppm of hexanes detected by gas chromatography.

### EXAMPLE 7

The lutein rich marigold oleoresin obtained in Example 3 and the zeaxanthin rich marigold oleoresin obtained in Example 4 are blended in the ratio of 15:85 by physical weight, respectively. 0.45 kg of blended marigold oleoresin containing 51.27g/kg of carotenoids (5.12% carotenoids) was homogenized for about 10 minutes in a 5.0 liters capacity 3 necked round bottom flask under continuous stirring at a temperature of 40° C in a hot water bath. Alcoholic KOH was prepared by taking 90 g of KOH (20% calculated to the input oleoresin) with the purity of around 90% and dissolving it in 675 ml (1:1.5 volumes to the oleoresin) of ethanol. The prepared alcoholic KOH was slowly added into reaction vessel containing the homogenized marigold oleoresin. The saponification reaction was carried out at a temperature of about 75°C for 30 minutes. After ensuring the degree of saponification to be more than 99% by HPLC, 1800ml of de-mineralized hot water (1:4 volumes is the ratio of the oleoresin to water which corresponds to about 1:1.4 volumes calculated to ratio of the reaction mixture to water) maintained at a temperature of about 75°C was added to the saponified marigold oleoresin and stirring was continued for about 10 minutes. The diluted saponified marigold oleoresin comprising carotenoid crystals was filtered in a buchner funnel to recover the carotenoid crystals. The carotenoid crystals thus obtained were washed with 3600 ml (1:8 volumes calculated to the oleoresin) of hot water maintained at around 75°C to remove the impurities and bring down the pH of the effluent to neutral around 7.0. The wet crystals were then collected from the filter and dried in a fluid bed drier at a temperature of around 55°C for 1 hour or until the moisture level is brought to less than 0.3% and the hexane and alcohol levels are less than 10 ppm and 100 ppm respectively.

13.13 g of purified carotenoids crystals rich in T-lutein and T-zeaxanthin were obtained and the physical recovery of the final product was 2.92%. The carotenoid crystals thus obtained contained 91.43% total carotenoids determined by UV-Visible spectrophotometer of which 47.45 % was all T-lutein, 46.25% all T-zeaxanthin, 0.85% beta-cryptoxanthin, and 4.28% of beta-carotene, 0.25% of cis-luteins and 0.25% of chrysanthemaxanthin, determined by HPLC.

The carotenoids crystals thus obtained contain 43.38% of T-lutein by weight and 42.28% by weight of T-zeaxanthin with the ratio of T-lutein to T-zeaxanthin by weight to be at about 1:1 ratio (1.02).

The carotenoid crystals thus obtained contained 8.52% of wax content when measured using gas chromatography. The carotenoids chemical recovery of the final product was 52.03% with a lutein chemical recovery of 58.3% and a zeaxanthin chemical recovery of 74.2%. The final product contained a moisture content of 0.30% with 15.38 ppm of ethanol and 8.4 ppm of hexanes detected by gas chromatography.

### EXAMPLE 8

80 kg of lutein rich marigold meal having 8.99 gm/kg (0.89%) of total carotenoids (with a carotenoids profile of 74.58% trans-lutein (T-lutein), 4.28% trans-zeaxanthin, 1.94% of beta-carotene, 1.02% of cryptoxanthin and 18.08% cis-isomers and epoxides measured by HPLC) was taken in a 1000 liters capacity extractor with circulation facility. 480 liters of Food grade hexane with a gas chromatographic purity of more than 98%, was added to the extractor and circulated. The temperature was raised to around 55°C and maintained for 1 hour, under circulation. After 1 hour the extract was drained and collected in a miscella tank. The extraction was repeated for two more times each with 480 liters of hexane under the same conditions of temperature and time. The extracts obtained were collected in the miscella tank. The fourth and fifth extractions were carried out using 400 liters of hexane in each extraction under circulation and at a temperature of about 58°C. 2084 liters of miscella collected from the five extractions was taken to an evaporator, preferably a falling film evaporator or a wiped film evaporator to obtain a concentrated miscella containing 70% solids and 30% solvent. The concentrated miscella was distilled further in a distillation unit under atmospheric pressure to increase the concentration of solids to 95% with a solvent level of around 5%. The solvent was distilled further under reduced pressure (450 to 600 mm Hg) to reduce the solvent level further to less than 1%. The total amount of solvent recovered was 1916 liters with a gas chromatographic purity of more than 98%. 6.728 kg of oleoresin containing 103.83 g/kg of carotenoids (10.38% carotenoids) was obtained with a carotenoids chemical recovery of 97.13%. The oleoresin thus obtained exhibited a carotenoids profile containing 73.9% trans-lutein, 5.89% T-zeaxanthin, 1.34% of beta-carotene, 0.52% of cryptoxanthin and 18.35% of cis-isomers/epoxides measured by HPLC.

6.3 kg of lutein rich marigold oleoresin obtained above containing 103.8 g/kg of carotenoids (10.38% carotenoids) was homogenized for 10 minutes in a 100 liters capacity reactor under continuous stirring at a temperature of about 40°C maintained by hot water circulation in the jacket. Alcoholic KOH was prepared by taking 1.26 kg of KOH (20% calculated to the input oleoresin) with the purity of around 90% and dissolving it in 9.4 liters (1:1.5 w/v to the oleoresin) of ethanol. The prepared alcoholic KOH was slowly added into reaction vessel containing the homogenized marigold oleoresin. The saponification reaction was carried out at a temperature of about 75°C for 30 minutes. After ensuring the degree of saponification to be more than 99% by HPLC, 25.2 liters of demineralized hot water (1:4 volumes is the ratio of the oleoresin to water which corresponds to about 1:1.4 volumes calculated to ratio of the reaction mixture to water) maintained at a temperature of 75°C was added to the saponified marigold oleoresin and stirring was continued for 10 minutes. The diluted saponified marigold oleoresin comprising carotenoid crystals was filtered through filter press to recover the carotenoid crystals. The carotenoid crystals thus obtained were washed with 50.4 Liters (1:8 w/v calculated to the oleoresin) of hot water maintained at around 75°C to remove the impurities and bring down the pH of the effluent to neutral around 7.0. The wet crystals were then collected from the filter and dried in a Fluid bed drier at a temperature of around 55°C for 1 hour or until the moisture level is brought to less than 0.3% and the hexane and alcohol levels are less than 10 ppm and 100 ppm respectively.

520.8 g of purified carotenoids crystals rich in T-lutein were obtained and the physical recovery of the final product was 8.26%. The carotenoid crystals thus obtained contained 90.72% total carotenoids determined by UV-Visible spectrophotometer of which 93.02% was all T-lutein, 6.21% all T-zeaxanthin, 0.18% of cis-luteins, 0.18 of betacarotene and 0.41% of cryptoxanthin determined by HPLC. The chemical recovery of the final product is 72.25% of Total carotenoids.

### EXAMPLE 9

20 kg of zeaxanthin rich marigold meal (obtained from Ball Horticulture Inc covered by the U.S. Patent No. 6,784,351) having 3.29 g/kg (0.329%) of total carotenoids with a carotenoid profile of 59.08% T-zeaxanthin, 14.80% of beta-carotene, 11.77% of T-lutein, 2.76% of alpha-cryptoxanthin, 2.33% of beta-cryptoxanthin, 1.98% of alpha-carotene, 0.82% of cis-lutein, 0.22% of chrysanthemaxanthin and 6.24% of other unidentified carotenoids, measured by HPLC was taken in a 200 liters capacity extractor with circulation facility. 120 liters of food grade hexane with a Gas chromatographic purity of more than 98%, was added to the extractor and circulated. The temperature was raised to about 45°C and maintained for 1 hour under circulation. After 1 hour, the extract was drained and collected in a miscella tank. The extraction was repeated for 3 more times with 60 liters of Hexane under the same conditions of temperature and time. The extracts obtained were collected in the miscella tank. 252 liters of miscella collected from the above 4 extractions was taken to an evaporator, preferably a falling film evaporator or a wiped film evaporator to obtain a concentrated miscella containing 70% solids and 30% solvent. The concentrated miscella was distilled further in a distillation unit under atmospheric pressure to increase the concentration of solids to 95% with a solvent level of around 5%. The solvent was distilled further under reduced pressure (450 to 600 mm Hg) to reduce the solvent level further to less than 1%. The total amount of solvent recovered was 224 liters with Gas chromatographic purity of more than 98%. 1.46 kg of oleoresin containing 42.88 g/kg of total carotenoids (4.28% of total carotenoids) was obtained with a recovery of 95.14%. The zeaxanthin chemical recovery was 87.74%. The oleoresin thus obtained exhibited a carotenoids profile containing 54.47% of T-zeaxanthin, 9.30% of beta-carotene, 19.96% of T-lutein, 3.23% of alpha-cryptoxanthin, 1.89% of beta-cryptoxanthin, 2.68% of alpha-carotene, 0% of cis-lutein and 0.12% of chrysanthemaxanthin and 8.35% of other unidentified carotenoids, measured by HPLC.

1.44 kg of zeaxanthin rich marigold oleoresin obtained above containing 42.88 g/kg of total carotenoids (4.28% of total carotenoids) was homogenized for around 10 minutes in a 10 liters capacity extractor under continuous stirring at a temperature of about 40°C maintained by hot water circulation in the jacket. Alcoholic KOH was prepared by taking 288 g of KOH (20% calculated to the input oleoresin) with the purity of around 90% and dissolving it in 2.16 liters (1:1.5 w/v to the oleoresin) of ethanol. The prepared alcoholic KOH was slowly added into reaction vessel containing the homogenized marigold oleoresin. The saponification reaction was carried out at a temperature of about 75°C for 30 minutes. After ensuring the degree of saponification to be more than 99% by HPLC, 5.76 liters of demineralized hot water (1:4 volumes is the ratio of the oleoresin to water which corresponds to about 1:1.4 volumes calculated to ratio of the reaction mixture to water) maintained at a temperature of about 75°C was added to the saponified marigold oleoresin and stirring was continued for 10 minutes. The diluted saponified marigold oleoresin comprising carotenoid crystals was filtered in a neutch filter to recover the carotenoid crystals. The carotenoid crystals thus obtained were washed with 11.5 liters (1:8 w/v calculated to the oleoresin) of hot water maintained at around 75°C to remove the impurities and bring down the pH of the effluent to neutral around 7.0. The wet crystals were then collected from the filter and dried in a fluid bed drier at a temperature of around 55°C for 1 hour or until the moisture level is brought to less than 0.3% and the hexane and alcohol levels are less than 10 ppm and 100 ppm respectively.

22.17 g of purified carotenoids crystals rich in T-zeaxanthin were obtained and the physical recovery of the final product was 1.54%. The carotenoid crystals thus obtained contained 75.01% total carotenoids determined by UV-Visible spectrophotometer of which 90.20% was all T-zeaxanthin, 6.32% all T-lutein, 0.10% of chrysanthemaxanthin, 0.62% of Alpha-carotene and 2.78% of beta-carotene and without any traces of cis-luteins and epoxides, determined by HPLC.

The carotenoids crystals thus obtained contain 67.66% of T-zeaxanthin by weight and 4.74% by weight of T-lutein with a chemical recovery of 26.98% of Total carotenoids.

### EXAMPLE 10

The obtained crystals from Example 8 and the crystals obtained from Example 9 are blended in specific ratios in the laboratory mixer blender for 10 minutes or until there is uniformity of color in the final blend. The blended sample is analyzed for its total carotenoids and HPLC profile. All the three blends are blended in the ratio of 10:1 1 T-lutein to T-zeaxanthin by taking different quantities at each blend. The theoretical profile of the 10:1 blend of T-lutein to T-zeaxanthin would be of 90.21% total carotenoids measured by UV with 90.2% T-lutein, 8.92% of T-zeaxanthin and with 0.86 % of other carotenoids, measured by HPLC.

Blend 1:240 g of lutein and 8 g of zeaxanthin is taken and blended as mentioned above. The carotenoid crystals thus obtained contained 90.26% total carotenoids determined by UV-Visible spectrophotometer of which 89.0% was all T-lutein, 9.82% all T-zeaxanthin and 1.18% of beta-carotene and other carotenoids without any traces of cis-luteins and epoxides, determined by HPLC. The obtained ratio in the above blend is 9.06.

Blend 2:180 g of lutein and 6 g of zeaxanthin is taken and blended as mentioned above. The carotenoid crystals obtained in blend 2 contained 90.63% total carotenoids determined by UV-Visible spectrophotometer of which 90.86% was all T-Lutein, 8.03% all T-zeaxanthin and 1.11% of beta-carotene and other carotenoids without any traces of cis-luteins and epoxides, determined by HPLC. The obtained ratio in the above blend is 11.31.

Blend 3:90g of lutein and 3g of zeaxanthin is taken and blended as mentioned above. The carotenoid crystals obtained in blend 3 contained 88.82% total carotenoids determined by UV-Visible spectrophotometer of which 90.61% was all T-lutein, 8.63% all T-zeaxanthin and 0.76% of beta-carotene and other carotenoids without any traces of cis-luteins and epoxides, determined by HPLC. The obtained ratio in the above blend is 10.49.

It is evident from the above experiment that the crystal blending after purifying individually results in varied ratios like 9.06, 11.31, and 10.49 when blended in the same ratios. It is also evident that the recovery of zeaxanthin purified crystals obtained above is very low as 26.98% which is not economical on a commercial scale.

Other modifications and variations to the invention will be apparent to those skilled in the art from the foregoing disclosure and teachings. Thus, while only certain embodiments of the invention have been specifically described herein, it will be apparent that numerous modifications may be made thereto without departing from the spirit and scope of the invention.

## Claims

1. Process for the isolation of carotenoid crystals having lutein and zeaxanthin in a weight ratio of 10:1, said process comprising:
contacting a plant source rich in lutein with hexane and extracting at a temperature of 40°C to 60°C to obtain an oleoresin rich in lutein;
contacting a plant source rich in zeaxanthin with hexane and extracting at a temperature of 40°C to 60°C to obtain an oleoresin rich in zeaxanthin;
mixing the oleoresin rich in lutein and the oleoresin rich in zeaxanthin in a ratio ranging from 80:20(w/w) to 90:10(w/w) and homogenizing to obtain a mixed oleoresin;
hydrolyzing the mixed oleoresin with an alcoholic alkali at a temperature of 70°C to 80°C to obtain a reaction mixture;
precipitating the carotenoid crystals by adding hot water to the reaction mixture to form a precipitate; and
obtaining the carotenoid crystals having lutein and zeaxanthin in a weight ratio of 10:1.

2. Process for the isolation of carotenoid crystals having lutein and zeaxanthin in a ratio of 5:1, said process comprising:
contacting a plant source rich in lutein with hexane and extracting at a temperature of 40°C to 60°C to obtain an oleoresin rich in lutein;
contacting a plant source rich in zeaxanthin with hexane and extracting at a temperature of 40°C to 60°C to obtain an oleoresin rich in zeaxanthin;
mixing the oleoresin rich in lutein and the oleoresin rich in zeaxanthin in a ratio ranging from 70:30(w/w) to 30:70(w/w) and homogenizing to obtain a mixed oleoresin;
hydrolyzing the mixed oleoresin with an alcoholic alkali at a temperature of 70°C to 80°C to obtain a reaction mixture;
precipitating the carotenoid crystals by adding hot water to the reaction mixture to form a precipitate; and
obtaining the carotenoid crystals having lutein and zeaxanthin in a weight ratio of 5:1.

3. Process for the isolation of carotenoid crystals having lutein and zeaxanthin in a ratio of 1:1, said process comprising:
contacting a plant source rich in lutein with hexane and extracting at a temperature of 40°C to 60°C to obtain an oleoresin rich in lutein;
contacting a plant source rich in zeaxanthin with hexane and extracting at a temperature of 40°C to 60°C to obtain an oleoresin rich in zeaxanthin;
mixing the oleoresin rich in lutein and the oleoresin rich in zeaxanthin in a ratio ranging from 10:90(w/w) to 20:80(w/w) and homogenizing to obtain a mixed oleoresin;
hydrolyzing the mixed oleoresin with an alcoholic alkali at a temperature of 70°C to 80°C to obtain a reaction mixture;
precipitating the carotenoid crystals by adding hot water to the reaction mixture to form a precipitate; and
obtaining the carotenoid crystals having lutein and zeaxanthin in a weight ratio of 1:1.

4. The process as claimed in any of claims 1 to 3, wherein the plant source rich in lutein and the plant source rich in zeaxanthin are independently selected from the group consisting of marigold flowers (*Tagetes erecta*)*,* marigold petals, mutant marigold flowers, mutated marigold petals, fruits of paprika, and berries of Chinese wolfberries (*Lycium barbarum*)*.*

5. The process as claimed in claim 4, wherein the plant source rich in lutein is selected from the group consisting of marigold flowers and marigold petals, and wherein the plant source rich in zeaxanthin is selected from the group consisting of mutant marigold flowers, mutated marigold petals, fruits of paprika, and berries of Chinese wolfberries.

6. The process as claimed in any of claims 1 to 5, wherein the alcoholic alkali is selected from the group consisting of ethanolic sodium hydroxide and ethanolic potassium hydroxide.

7. The process as claimed in any of claims 1 to 6 wherein the alcoholic alkali is 10%-30%.

8. The process as claimed in any of claims 1 to 7, wherein ratio of the reaction mixture to hot water is in the range of about 1:1 to 1:1.5 (v/v).

9. Carotenoid crystals having lutein and zeaxanthin in a weight ratio of 10:1, made by a process comprising:
contacting a plant source rich in lutein with hexane and extracting at a temperature of 40°C to 60°C to obtain an oleoresin rich in lutein;
contacting a plant source rich in zeaxanthin with hexane and extracting at a temperature of 40°C to 60°C to obtain an oleoresin rich in zeaxanthin;
mixing the oleoresin rich in lutein and the oleoresin rich in zeaxanthin in a ratio ranging from 80:20(w/w) to 90:10(w/w) and homogenizing to obtain a mixed oleoresin;
hydrolyzing the mixed oleoresin with an alcoholic alkali at a temperature of 70°C to 80°C to obtain a reaction mixture;
precipitating the carotenoid crystals by adding hot water to the reaction mixture to form a precipitate; and
obtaining the carotenoid crystals having lutein and zeaxanthin in a weight ratio of 10:1.

10. Carotenoid crystals having lutein and zeaxanthin in a weight ratio of 5:1, made by a process comprising:
contacting a plant source rich in lutein with hexane and extracting at a temperature of 40°C to 60°C to obtain an oleoresin rich in lutein;
contacting a plant source rich in zeaxanthin with hexane and extracting at a temperature of 40°C to 60°C to obtain an oleoresin rich in zeaxanthin;
mixing the oleoresin rich in lutein and the oleoresin rich in zeaxanthin in a ratio ranging from 70:30(w/w) to 30:70(w/w) and homogenizing to obtain a mixed oleoresin;
hydrolyzing the mixed oleoresin with an alcoholic alkali at a temperature of 70°C to 80°C to obtain a reaction mixture;
precipitating the carotenoid crystals by adding hot water to the reaction mixture to form a precipitate; and
obtaining the carotenoid crystals having lutein and zeaxanthin in a weight ratio of 5:1.

11. Carotenoid crystals having lutein and zeaxanthin in a weight ratio of 1:1, made by a process comprising:
contacting a plant source rich in lutein with hexane and extracting at a temperature of 40°C to 60°C to obtain an oleoresin rich in lutein;
contacting a plant source rich in zeaxanthin with hexane and extracting at a temperature of 40°C to 60°C to obtain an oleoresin rich in zeaxanthin;
mixing the oleoresin rich in lutein and the oleoresin rich in zeaxanthin in a ratio ranging from 10:90(w/w) to 20:80(w/w) and homogenizing to obtain a mixed oleoresin;
hydrolyzing the mixed oleoresin with an alcoholic alkali at a temperature of 70°C to 80°C to obtain a reaction mixture;
precipitating the carotenoid crystals by adding hot water to the reaction mixture to form a precipitate; and
obtaining the carotenoid crystals having lutein and zeaxanthin in a weight ratio of 1:1.

12. Carotenoid crystals comprising:
lutein and zeaxanthin, wherein a weight ratio of lutein to zeaxanthin is 10:1.

13. Carotenoid crystals comprising:
lutein and zeaxanthin, wherein a weight ratio of lutein to zeaxanthin is 5:1.

14. Carotenoid crystals comprising:
lutein and zeaxanthin, wherein a weight ratio of lutein to zeaxanthin is 1:1.

15. Use of carotenoid crystals comprising a weight ratio of lutein to zeaxanthin of 10:1 or of 5:1 or of 1:1 as antioxidants.
